# EUROPEAN PATENT SPECIFICATION

(11) **EP 2 046 786 B1**
(45) Date of publication and mention of the grant of the patent: **27.12.2017**
(21) Application number: 07786532.7
(22) Date of filing: 02.08.2007
(51) Int. Cl.: C07D 417/12, C07D 275/04, A61K 31/427

(54) **POLYMORPHIC FORMS OF ZIPRASIDONE SULFATES**
POLYMORPHE FORMEN VON ZIPRASIDONSULFATEN
FORMES POLYMORPHIQUES DE SULFATES DE ZIPRASIDONE

(30) Priority: 02.08.2006 EP 06016132; 15.05.2007 EP 07009715
(43) Date of publication of application: 15.04.2009
(73) Proprietor: KRKA, 8501 Novo mesto (SI)
(72) Inventor: RUZIC, Milos, 3000 Celje (SI); SMRKOLJ, Matej, Zagorje ob Savi (SI); PECAVAR, Anica, 8000 Novo mesto (SI); STERGAR, Matej, 3000 Celje (SI); MAKUC, Simon, 8000 Novo mesto (SI); JURSIC, Urska, 8322 Stopice (SI); ZAJC, Natalija, 8340 Crnomelj (SI); ZADNIK, Jernej, 8350 Dolenjske Toplice (SI); HVALEC, Miran, 8275 Skocjan (SI)
(74) Representative: Becker Kurig Straus
(86) International application number: PCT/EP2007/006862
(87) International publication number: WO 2008/015005

(56) References cited:
- EP-A2- 0 965 343
- WO-A-2005/065660
- WO-A-2006/034964

## Description

### Field of the invention

The present invention relates to novel polymorphic forms of Ziprasidone sulfates, as well as to a process for their preparation and pharmaceutical formulations containing it. The present polymorphic forms of Ziprasidone sulfates are characterized by small average particle sizes and high solubility bestowing the compounds an improved bioavilability in comparision to up to now available Ziprasidone salts.

### Background of the invention

Ziprasidone is the common name for 5-[2-[4-(1,2-benzisothiazol-3-yl)-1-piperazinyl]ethyl]-6-chloro-1, 3-dihydro-2H-indol-2-one, of the formula : 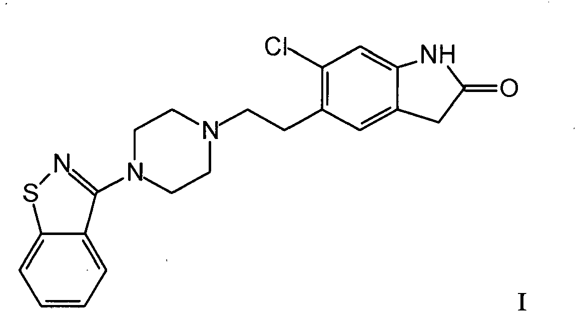 an active pharmaceutical ingredient having neuroleptic activity.

EP 0 281 309 decribes a process for the preparation of Ziprasidone by means of a coupling reaction performed in organic polar solvents, such as ethanol, N,N-dimethylformamide (DMF) and methylisobutylketone (MIBK). In Example 16 of EP 0 281 309-A1 a process for preparing Ziprasidone hydrochloride in hemihydrated form is disclosed.

EP 0 584 903 discloses an alternative approach for the preparation of Ziprasidone, Ziprasidone salts including hydrochloride and methanesulfonate, wherein the coupling reaction is carried out in water in the presence of an excess of sodium carbonate as neutralizing agent.

According to EP 0 281 309, EP 0 584 903 and EP 1 029 861, pharmaceutically acceptable acid addition salts of the Ziprasidone may be prepared in a conventional manner by treating a solution or suspension of the Ziprasidone free base with about one chemical equivalent of a pharmaceutically acceptable acid. Conventional concentration and recrystallization techniques are employed in isolating the salts.

EP 0 918 772 and EP 0 904 273 refer to dihydrate and trihydrate salts of Ziprasidone mesy-late which are reported to possess increased hygroscopic stability and increased aqueous solubility than the hydrochloride salts.

In WO2006/034964 a process of using organic polar solvents, such as acetonitrile, is disclosed for the preparation of Ziprasidone pharmaceutically acceptable acid addition salt, such as maleate or acetate.

WO 2005/065660 and EP 0 965 343 disclose Ziprasidone formulations and in particular Ziprasidone hydrochloride monophosphate. Ziprasidone hydrogensulfate and Ziprasidone sulfate dihydrates are not disclosed.

In view of the prior art there still exists a need for an improved method for the preparation of Ziprasidone salts.

### Object of the invention

An object of the present invention resides in the provision of Ziprasidone salts with defined properties rendering them more suitable for the preparation of pharmaceutical compositions. Another object of the present invention resides in the provision of Ziprasidone salts, which may be better tolerated if ingested.

The present invention solves the above-mentioned problems by the provision of Ziprasidone sulfates, i.e. Ziprasidone sulfate and Ziprasidone hydrogensulfate, having specific amounts of crystal water. The present Ziprasidone sulfates may be obtained in high purity, have good stability and advantageous formulation properties particularly in comparison to the hydrochloride form of Ziprasidone. The present inventors have surprisingly found out that the present compounds provide improved properties with respect to solubility and particle size. The solubility of the compounds is on the one hand directly influenced by the content of crystal water and on the other hand by the low particle size. Said effects in turn influence tolerance and incorporation of said compounds upon ingestion, since smaller amounts of the drugs may be employed in respective pharmaceutical compositions exhibiting anyway the same or even improved bioavailability in comparison to compounds of the state of the art.

### Figures

In the figures are, where applicable, (a) table , (b) FT-IR spectra and (c) microscopic picture.

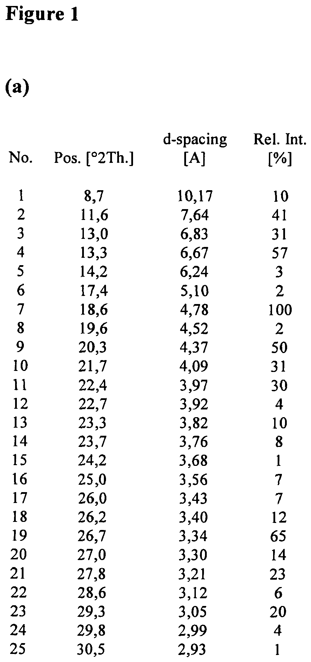

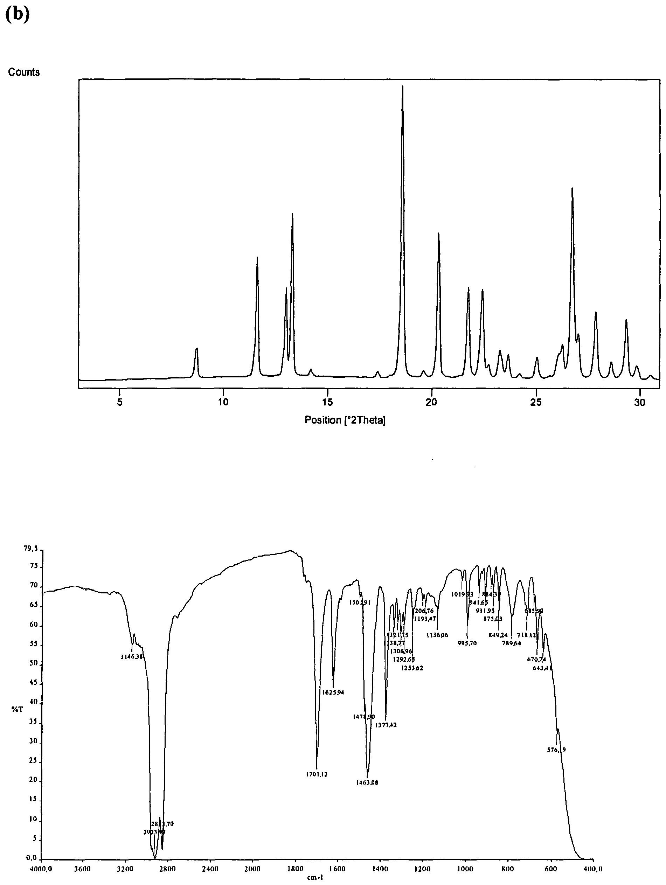

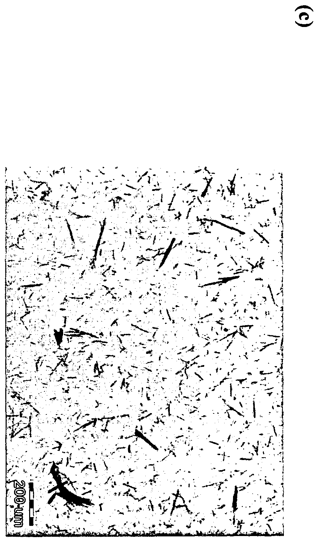

Figure 1 shows an X-ray powder diffraction spectrum of 6-Chloro-5-(2-Chloroethyl)-1,3-Dihydro-2H-Indol-2-One (INT 1).

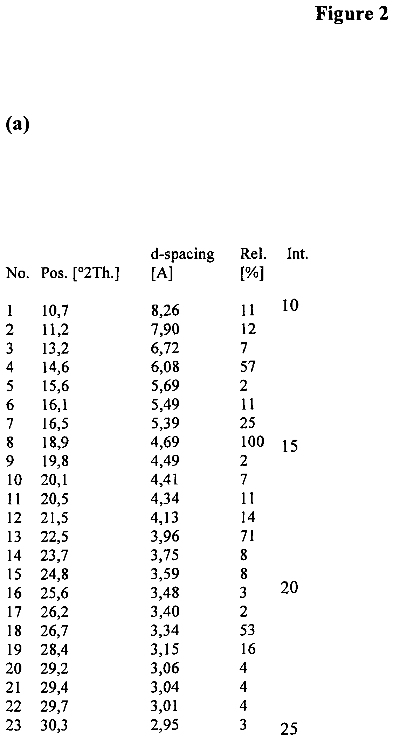

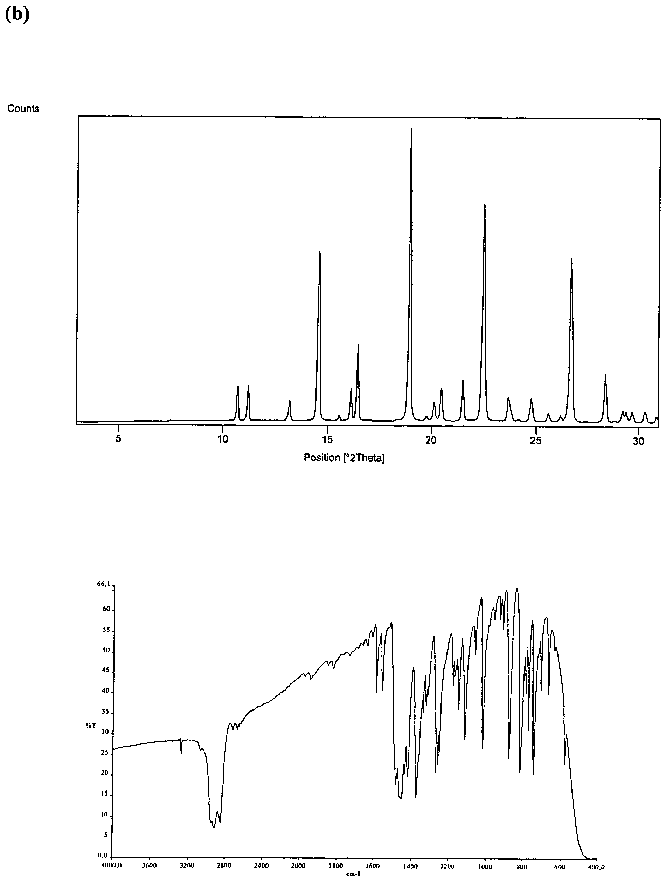

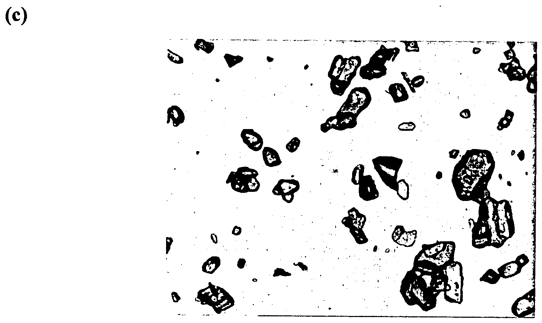

Figure 2 shows an X-ray powder diffraction spectrum of (3-(1-piperazinyl)1,2-benzisothiazole) (INT 2).

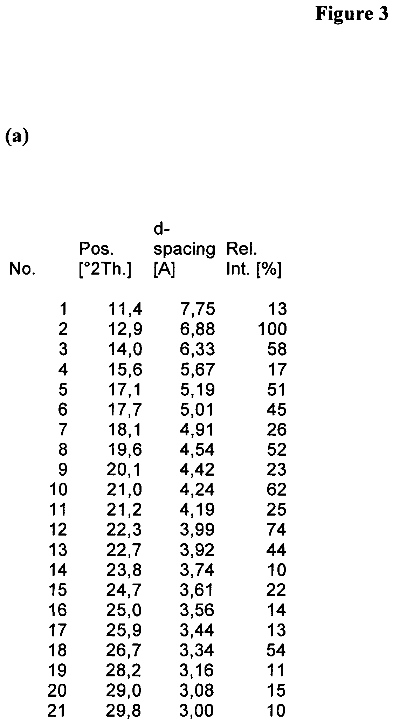

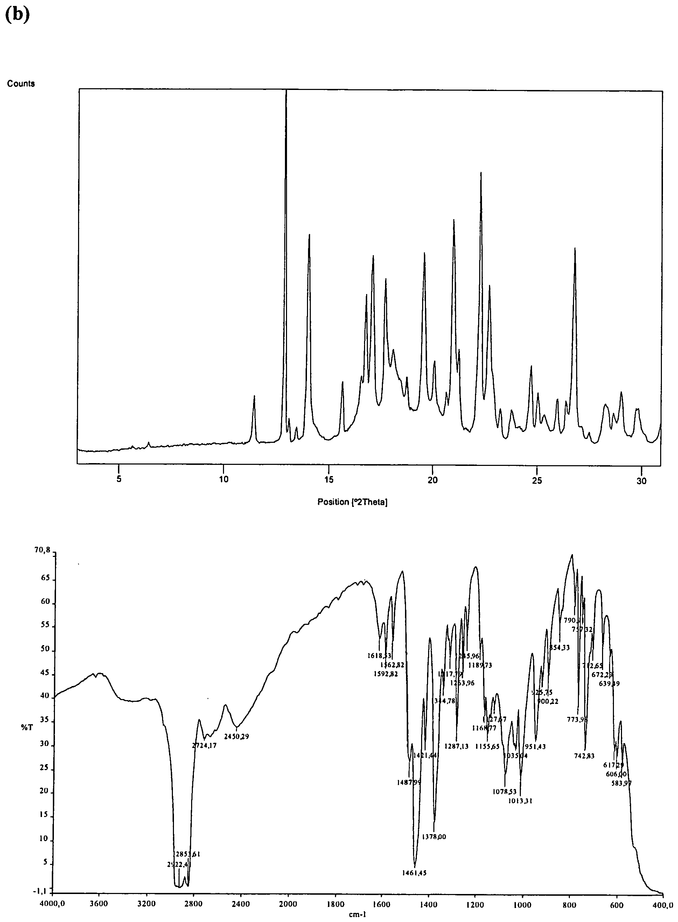

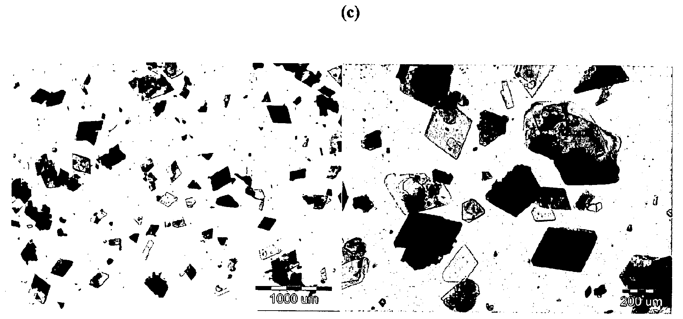

Figure 3 shows an X-ray powder diffraction spectrum of (3-(1-piperazinyl)1,2-benziso-thiazole) sulfate hydrate (INT 2 sulfate hydrate).
Figure 4 shows an X-ray powder diffraction spectrum of 5-[2-[4-(1,2-benzisothiazol-3-yl) -1-piperazinyl] ethyl] -6-chloro-1, 3- dihydro-2H-indol-2-one hydrogensulfate dihydrate (Ziprasidone hydrogensulfate dihydrate).
Figure 5 shows an X-ray powder diffraction spectrum of 5-[2-[4-(1,2-benzisothiazol-3-yl)-1-piperazinyl] ethyl] -6-chloro-1, 3- dihydro-2H-indol-2-one hydrogensulfate anhydrous.

### Detailed description of the invention

According to a first embodiment of the present invention a compound with formula I, a Ziprasidone sulfate or Ziprasidone hydrogensulfate, is provided having the general formula:

[Ziprasidone * H]ₙ⁺ [X] * Z H₂O

wherein
Ziprasidone is a compound of the formula; 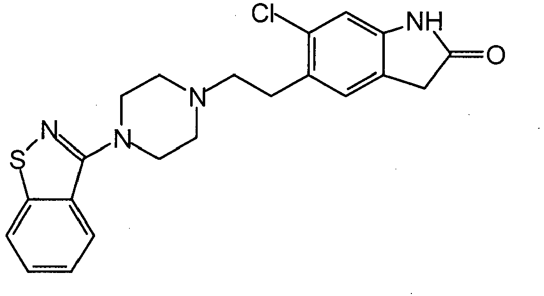
H is hydrogen;
n is 1 or 2;
X is HSO₄⁻ or SO₄²⁻; and
Z is 2.

Z<0.28 designates hereby the essential water free or anhydrous compound I with an approximate water content below 1 % per weight.

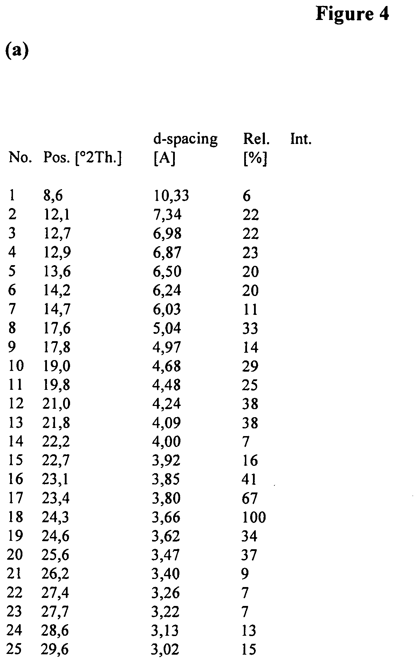

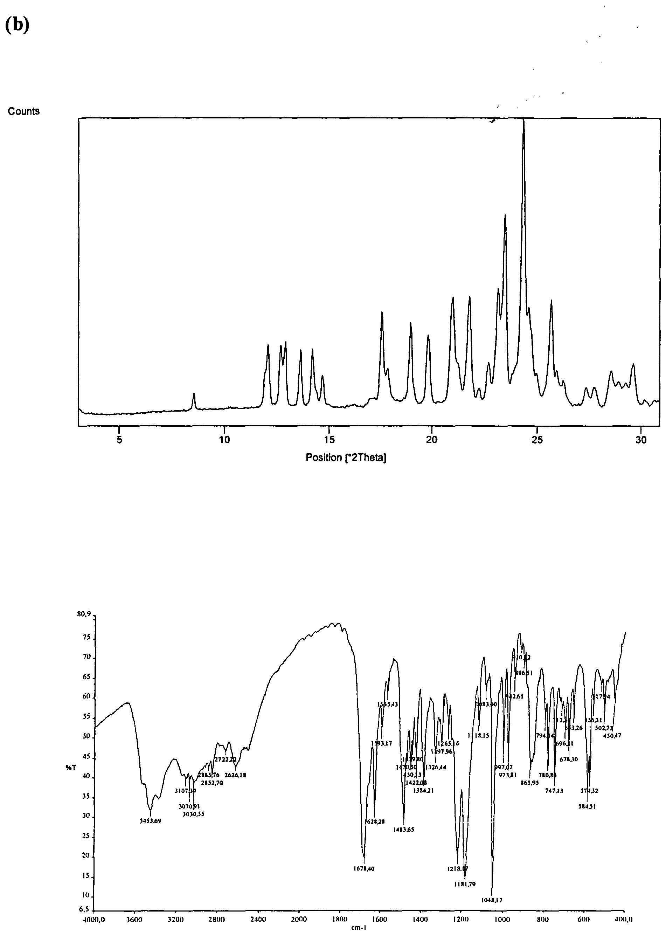

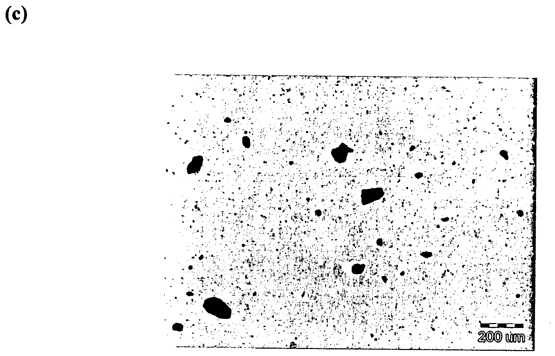

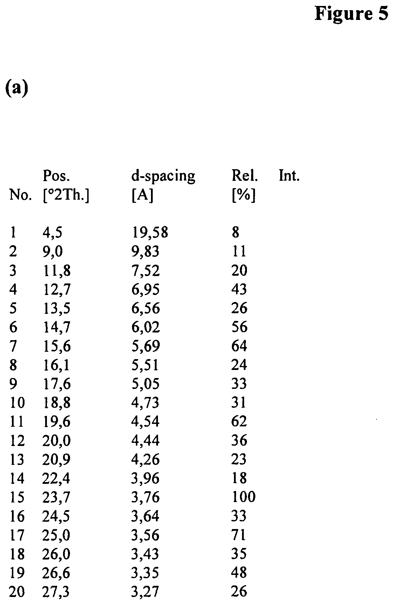

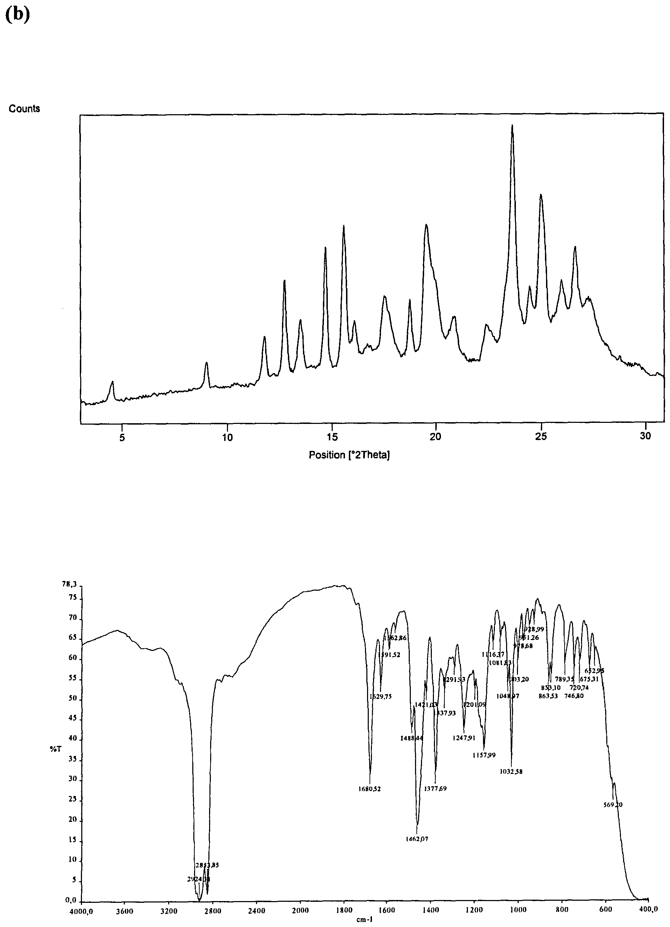

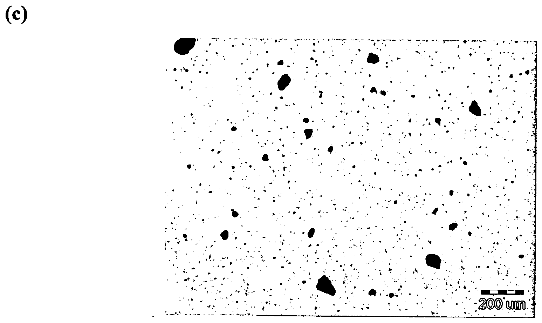

Preferably, the compound is Ziprasidone hydrogensulfate dihydrate.
According to an embodiment, the present compound has a particle size in the range of about 5 µm to about 300 µm, preferably in the range of about 10 µm to about 150 µm and more preferably in the range of about 20 µm to about 80 µm.
The average particle size or particle size referring to the volume mean diameter of particles of the present compounds may be determined by any suitable method known to the skilled person. Examples for suitable methods are based for example on sedimentation, by laser light scattering laser diffraction methods or electrical differential mobility analysis. Preferably, laser light scattering is employed.
In another embodiment, the compound according to the present invention has a solubility in the range of about 50 mg/l to about 800 mg/l, preferably in the range of about 100 mg/l to about 500 mg/l and more preferably in the range of about 250 mg/l to about 400 mg/l.
Different methods for detecting the water content known to the skilled person may be used such as the method according to Karl Fischer, which is described inter alia in European Pharmacopeia 5th edition 2006; Method 2.5.12.
Both small average particle size and high solubility favour a high bioavailability - representing a measure of the rate and extent of a therapeutically active drug that reaches the systemic circulation and which is available at the site of action - of the present compound.
According to another embodiment, the present compounds are characterized by a X-ray powder diffraction pattern exhibiting characteristic diffractions at 8,6; 12,1; 13,6; 17,6; 19,8; 23,4 in 24,3° ± 0,2°2Theta (Form I) or 4,5; 9,0; 15,6; 23,7; 25,0 in 26,6° ± 0,2°2Theta (Form II). Preferably, the Form I and II are characterized by a X-ray powder diffraction pattern as outlined in Figure 4 and Figure 5, respectively. X-ray powder diffraction patterns are particularly suitable to describe polymorphic forms.

The discovery of new polymorphic forms of a pharmaceutically useful compound provides a new opportunity to improve the performance characteristics of a pharmaceutical product. It enlarges the repertoire of materials that a formulation scientist has available for designing, for example, a pharmaceutical dosage form of a drug with a targeted release profile or other desired characteristic.

According to another preferred embodiment of the present invention, a process for the preparation of compounds according to the present invention is provided. The process comprises the steps of (i) providing Ziprasidone base, (ii) reacting Ziprasidone base with defined amounts of both water and sulfuric acid, (iii) optionally cooling and/or drying, and (iv) obtaining compound of formula I having a defined water content, characterized by specific crystal structures.

It will be appreciated that alternatively in a first step also water and/or sulfuric acid may be provided and subsequently reacted with the remaining educt(s). Alternatively, instead of Ziprasidone base a mixture of Ziprasidone base and a Ziprasidone salt may be used.

Alternatively, any other reaction(s) yielding e.g. crude Ziprasidone hydrochloride, which are for example outlined in EP 0 281 309, EP 0 584 903, EP 1 029 861, WO2006/034964, WO2006/034965, WO2006/011157, WO2006/047893, WO2005/085240, US2006/0089502, may be used. Such compounds may be in turn converted to Ziprasidone sulfates by methods known to the skilled person.

The respective anhydrous compound I, characterized by Z≤0.28, may be obtained by standard drying procedures for removing solvents. Examples for suitable drying procedures comprise application of low pressure or desiccator containing one or more chemicals suitable for removing solvent by e.g. binding to them.

In order to obtain the desired product, i.e. Ziprasidone sulfate with a defined water content or the anhydrous compound is reacted with defined amounts of sulfuric acid and water. Alternatively, an excess of water may be employed followed by a drying step, e.g. by means of an air dryer or any other drying method as outlined above, to the desired extend (as determined e.g. by the method according to Karl Fischer).
It should be appreciated that sulfuric acid of any water content or essential water free sulfuric acid or even oleum may be used for the above mentioned conversion to the desired compound. In such cases the water content of the sulfuric acid may be considered and incorporated in calculating the ratio of sulfuric acid and water used.
Any kind of ratio of Ziprasidone base, sulfuric acid and water may be used for performing the reaction.
Duration and temperature of the reaction may be chosen according to common knowledge. Preferably the reaction is conduced for 1-6 hours at temperatures in the range of 20-80°C. More preferably, the reaction is conduced first at ambient temperature, i.e. in a range between 20 and 25°C, for 10-120 minutes and than heated to 50-70°C for other 2-5 hours. After performing the reaction the mixture obtained may be optionally cooled, filtered and/or dried.

Ziprasidone base or crude Ziprasidone is prepared by reacting 6-Chloro-5-(2-chloroethyl)-1,3-dihydro-2H-indol-2-one (INT 1) and (3-(1-piperazinyl)1,2-benzisothiazole) hydrogen sulfate (INT 2) in presence of an alkaline compound; and an organic solvent, optionally mixed with water. The organic solvent is preferably selected from the group of ionic liquids.
Ionic liquids can be selected from the group 1-R-3-R1-imidazolium H(+) x A(-), wherein R and R1 are lower alkyl and A(-) are mono- or polyprotic anion of acid. The term lower alkyl is directed to C₁ to C₁₀ hydrocarbons, which may comprise linear and/or branched chaines, saturated and/or insaturared atoms, and heteroatoms.

Examples for sutible ionic liquids are 1,2,3-Trimethylimidazolium methylsulfate, 1-Butyl-2,3-dimethylimidazolium chloride, 1-Butyl-3-methylimidazolium acetate, 1-butyl-3-methylimidazolium bromide, 1-Butyl-3-methylimidazolium chloride, 1-Butyl-3-methylimidazolium hexafluorophosphate, 1-Butyl-3-methylimidazolium hydrogensulfate, 1-Butyl-3-methylimidazolium methansulfonate, 1-Butyl-3-methylimidazolium methylsulfate, 1-Butyl-3-methylimidazolium tetrachloroaluminate, 1-Butyl-3-methylimidazolium tetrafluoroborate, 1-Butyl-3-methylimidazolium thiocyanate, 1-Ethyl-2,3-dimethylimidazolium ethylsulfate, 1-Ethyl-3-methylimidazolium acetate, 1-Ethyl-3-methylimidazolium chloride, 1-Ethyl-3-methylimidazolium ethylsulfate, 1-Ethyl-3-methylimidazolium hydrogensulfate, 1-Ethyl-3-methylimidazolium methanesulfonate, 1-Ethyl-3-methylimidazolium methylsulfate, 1-Ethyl-3-methylimidazolium tetrachloroaluminate, 1-Ethyl-3-methylimidazolium tetrafluoroborate, 1-Ethyl-3-methylimidazolium thiocyanate, 1-Hexyl-3-methylimidazolium tetrafluoroborate, 1-Hexyl-3-methylimidazolium tetrafluoroborate, 1-Hexyl-3-methylimidazolium trifluoromethanesulfonate, 1-Methyl-3-octylimidazolium hexafluorophosphate, 1-Methyl-3-octyl-imidazolium trifluoromethanesulfonate, Methylimidazolium chloride, Methylimidazolium hydrogensulfate, Methyl-tri-n-butylammonium methylsulfate. Preferably 1-Ethyl-3-methylimidazolium ethylsulfate or 1-Ethyl-3-methylimidazolium methylsulfate are employed. Ionic liquids may be obtained for example by Merck, Germany. Also mixtures of one or more ionic liquids with water can be used.

From the above mentioned ionic liquids, particularly 1-Ethyl-3-methylimidazolium methyl sulfate and 1-Ethyl-3-methylimidazolium ethylsulfate proved to be particularly suitable for the preparation of the present compounds due to a good solubility of the educts.

Preferably, Na₂CO₃ and 1-Ethyl-3-methylimidazolium methyl sulfate are employed. Respective amounts of the compounds, reaction temperature and duration are known to the skilled person.

Preferably, the reaction is performed at a temperature ranging from room temperature to reflux temperature of the solvent applied, more preferably at the temperature selected from 80°C to 120°C. Preferably, the reaction is kept at the selected temperature for 3 to 80 h, more preferably for 10 to 30 h. The term "room temperature" as used herein refers to a temperature of about 22°C.

The Ziprasidone base can also be prepared by reduction of 5-[2-[4- (1,2-benzisothiazol-3-yl)-1-piperazinyl]-1-oxoethyl]-6-chloro-1,3-dihydro-2H-indol-2-one ("oxoethyl-Ziprasidone") or addition salts thereof.

After the completion of the reaction the reaction mixture is preferably cooled to room temperature and the product is precipitated by adding water or organic solvent or mixtures thereof. The organic solvents can be selected from C₁ - C₅ alcohol, acetonitrile or tetrahydrofuran, acetic acid, acetone and/or ethers. More preferably alcohols are selected from methanol, ethanol and isopropanol and ether is selected from isopropyl ether.

The ratio of added water or organic solvent or mixtures thereof with regard to initial amount of 3-(1-piperazinyl)1,2-benzisothiazole is in the range from 1 : 1 to 100 : 1, preferably 3 : 1 to 50 : 1, and more preferably 5 : 1 to 20 : 1 (g/g).

The Ziprasidone base may further be purified either by hot maceration or precipitation in the above mentioned solvents. Both purification methods are well known to the skilled person. Prior to formation of salt, Ziprasidone base is optionally pre-granulated.

Ziprasidone sulfates can also be prepared by mixing a Ziprasidone base or a mixture of Ziprasidone base and Ziprasidone salt with an organic or inorganic acid in the above mentioned solvents or water or mixtures thereof. The temperature may range from the room, i.e. 22°C, to reflux temperature of the solvent applied, preferably at a temperature ranging between 50°C to 60 °C. Afterwards Ziprasidone (hydrogen)sulfate hydrate is isolated/obtained.

The inorganic acids are the same as outlined above. Suitable other inorganic acids and organic acids are known to the skilled person. Solvents, Ziprasidone base as well as a suitable Ziprasidone salt may be provided and used as outlined above.

The ratio of mixed ziprasidone base or a mixture of Ziprasidone base and Ziprasidone salt and solvents or water or mixtures thereof is in the range 1 to 1 (g of ziprasidone base to ml of solvents or water) to 1 to 1000, preferably 1 : 5 to 1 : 100, and more preferably 1 : 7 to 1 : 15.

The ratio of mixed ziprasidone base or a mixture of Ziprasidone base and Ziprasidone salt and organic or inorganic acid is in the range 1 to 0,01 to 1 to 100 (g of ziprasidone base to g of base), preferably 1 : 0,1 to 1 : 10, and more preferably 1 : 0,2 to 1 : 0,5.

The amount of ziprasidone can be mixed as a whole amount in one step or it can be added in divided portions.

Ziprasidone sulfates may be prepared by mixing with solvent or base or mixtures thereof in any order. Preferably, Ziprasidone base and Ziprasidone salt is mixed with solvent and acid. A base can be added to the mixture of ziprasidone and solvent as outlined in the examples.

The solution can be filtered and then cooled to a temperature between -10 and 30°C, preferably to room temperature.

The precipitate is filtered and the product is dried preferably at a temperature between 10°C and 60°C, more preferably between 40 °C and 50°C. Drying may be achieved e.g. via application of heat, preferably carried out under ambient or reduced pressure or via contacting a Ziprasidone salt with humid air in a fluidized bed drier, wherein the atmosphere in the fluidized bed drier is at least 15% humidity. Preferably, air drying in a commercially available humid air dryer is applied.

According to a preferred embodiment, the present compounds are used for the preparation of a pharmaceutical composition for the prevention and/or treatment of psychotic states, preferably schizophrenia, schizoaffective disorder, delusional disorder, bipolar disorder, psychotic depression, brief psychotic disorder and anxiety diseases.

Suitable pharmaceutical compositions comprise preliminary enteral and parenteral Pharmaceutical compositions employing respective carriers in different amounts.

The pharmaceutical compositions may be in a solid dosage form. Exemplary solid dosage forms include tablets, capsules, sachets, lozenges, powders, pills or granules. The solid dosage form may be, for example, immediate release dosage form, a fast melt dosage form, controlled release dosage form, lyophilized dosage form, delayed release dosage form, extended release dosage form, pulsatile release dosage form, mixed immediate release and controlled release dosage form, or a combination thereof. A solid dose tablet formulation is preferred. The solid dosage form is preferably an immediate release dosage form offering advantages regarding the bioavailability of the active compound.

The dosage form may produced in accordance with usual techniques in which the active ingredient or substance and one or more are mixed and granulated by adding solvent in a low or high shear mixer or by fluidized bed granulator. The granulate is dried, for example in a fluidized bed dryer. The dried granulate is sized. The sizing of the micromatrix particles may be performed by using an oscillating granulator, comminuting mill or any other conventional method. The sieve used for the sizing may have openings from 0.25 mm to 5 mm. Alternatively, the core particles can be made by extrusion, spheronization, melt granulation or by roller compaction. The core particles may be coated by a solution of one or more release controlling agents by any known method, including spray application. Spraying can be carried out using a fluidized bed coater (preferably Wurster coating), or in a pan coating system. Alternatively the coating of the core particles with one or more rate controlling agents can be done by hot melt process using a granulator or fluidized bed coater (preferably Wurster coating), or in a pan coating system. The compression of micro tablets is carried out on usual compression machines (e. g. machines by Manesty, Cadmach or Kilian). The micro tablets can be made of various sizes and shapes like round, oval, oblong, capsule shaped, triangular, square, etc. The preferred shape of the micro tablet is round, biconvex and the preferred diameter of the micro tablet is 1.5 mm to 9.5 mm.

The micro tablets may be coated by a solution of one or more release controlling agents by any known method, including spray application. Spraying can be carried out using a fluidized bed coated (preferably Wurster coating), or in a pan coating system.

The present composition may by also present in a particular dosage form for further improving the bioavailability of the present compound. The bioavailability of orally ingested drugs is determined by factors, which include the nature of the molecule, its stability, and the formulation administered - and in the patient - such as a reduced intestinal surface area as a result of colic disease or intestinal resection and whether or not the drug is taken with a meal. Factors influencing the bioavailability may include, but are not limited to a poor absorption from the gastrointestinal tract, hepatic first-pass effect and degradation of the drug prior to reaching system circulation.

For improving bioavailability of the present compound, e.g. the particle size fractions may be adapted by further crushing. Preferable particle size after crushing is d₉₀, i.e. the average particle diameter of 90% of the particles, less than 30 µm, more preferably less than 20 µm, most preferably less than 10 µm.

The present pharmaceutical composition may contain in addition to the present compounds one or more diluents, binding agents, disintegrants, lubricants, sweeteners, glidants, flavourings, colouring agents and other excipients, depending on the dosage form desired.

Suitable diluents include pharmaceutically acceptable fillers such as lactose, microcrystalline cellulose, dibasic calcium phosphate, saccharides and/or mixtures of the foregoing. Examples of diluents include microcrystalline cellulose, such as Avicel PH 101® and Avicel® PH 102; lactose such as lactose monohydrate, lactose anhydrous and Pharmatose® DCL 21; dibasic calcium phosphate such as Emcompress®; mannitol, starch, sorbitol, sucrose and glucose. The most preferred are microcrystalline cellulose and lactose.

Binding agents are preferably selected from polyvinylpyrolidone, starch grades (pre-gellatinized or plain), cellulose derivatives such as hydroxypropylmethylcellulose (HPMC), hydroxypropylcellulose (HPC) and carboxymethylcellulose (CMC) and their salts and gelatine, the most preferred is HPMC.

Suitable disintegrants include croscarmellose sodium, crospovidone, sodium starch glycolate, corn starch, potato starch, maize starch and modified starches calcium silicates, low substituted hydroxypropylcellulose and the like. Most preferred is croscarmellose sodium.

Lubricants are preferably selected from the group consisted of magnesium stearate, magnesium lauryl sulfate and sodium stearyl fumarate, sucrose esters or fatty acid, polyethylene glycol, stearic acid and the like.

Sweeteners are preferably selected from the group consisting of aspartame, saccharin sodium, dipotassium glycyrrhizinate, aspartame, stevia, thaumatin, and the like.

Glidants are preferably selected from the group consisting of silicon dioxide, talc and aluminium silicate.

As flavourings, colouring agents, or opacifying agents and pigments any suitable compound known to the skilled person may be used.

Compositions suitable for parenteral administration comprise sterile aqueous and non-aqueous injection solutions of the active compound, which preparations are preferably isotonic with the blood of the intended recipient.

These preparations may contain anti-oxidants, buffers, bacteriostats and solutes, which render the compositions isotonic with the blood of the intended recipient. Aqueous and non-aqueous sterile suspensions may include suspending agents and thickening agents. The compositions may be presented in unit-dose or multi-dose containers, for example sealed ampoules and vials, and may be stored in a freeze-dried or a lyophilized condition requiring only the addition of the sterile liquid carrier, for example, saline or water-for-injection immediately prior to use. Extemporaneous injection solutions and suspensions may be prepared from sterile powders, granules and tablets of the kind previously described.

The concentration of active compounds in the present composition will depend on e.g. absorption, inactivation, excretion rates, the dosage schedule and amount administered as well as other factors known to those of skilled in the art. Other ingredients to produce e.g. an enteral applicable composition, such as a tablet or capsule or another suitable form, are also well known to the skilled person and may be formulated depending on the desired consistency and other properties envisaged.

The following examples illustrate the invention without limiting it thereto.

### Examples

The compounds according to the present invention were characterized with regard to their meltings points by means of a Koffler melting point apparatus, IR spectra taken on a Paragon 100 Perkin-Elmer FT-IR spectrometer (performed on a FT-IR System SPECTRUM GX Perkin Elmer [4000-400] cm⁻¹ at a resolution 4 cm⁻¹, preparing KBr tbl.) and X-ray powder diffraction patterns (obtained by a Phillips PW3040/60 X'Pert PRO diffractometer using CuK_{α} radiation). Water contents of the respective compounds have been tested by the method according to Karl Fischer. Images of particles were taken on a Microscope Olympus BX 50 equipped with Olympus camera DP70.

A high resolution HPLC method is used to determine an amount and purity of INT1, INT 2, oxoethyl-Ziprasidone and Ziprasidone base. The tests are carried out in Zorbax Eclipse XDB-C18, 1,8 µm, 50 x 4,6 mm column and X-bridge C18, 2.5 µm, 75 x 4.6 mm column. The mobile phase is gradient of 0.01 M diammonium hydrogenphosphate, pH 3 and acetonitrile /methanol (20/80 ratio). The chromatograph is equipped with a UV detector set at 230 nm, the flow rate is 1.0 ml per minute at 40°C.

The average particle size is determined by laser light scattering using a Malvern-Mastersizer Apparatus MS 2000. The particles to be subjected to the particle size measurement are first suspended in isoparafinic oil (Isopar®) and then subjected to a size determination in a Malvern Mastersizer MS 2000 instrument. Usually, 100-800 mg of substance is dispersed in 5-8 ml of vegetable oil. According to manufacturer's information, the Malvern Mastersizers allow for a measurement of particle size distributions in the range of 20 nm to 2000 µm, with a precision of better than 0.5%. Particle sizes are determined by measuring the angular distribution of laser light scattered by a homogeneous suspension of particles. The size distribution is determined from the light scattering data using the theory of light scattering developed by Gustav Mie.

### Example 1

### Preparation of (3-(1-piperazinyl)1,2-benzisothiazole) sulfate hydrate (INT 2 sulfate)

An amount of 1 g of 3-(1-piperazinyl)1,2-benzisothiazole (INT2), 10 ml of demineralized water and 1.9 g of H₂SO₄ (INT2 : H₂SO₄ ratio is 1:5) were mixed at room temperature overnight. After the completion of the process clear solution became white. The product was filtered and dried for 2 hours at 30 °C. An amount of 0.3 of white precipitate of 3-(1-piperazinyl)-1,2-benzisothiazole sulfate hydrate (INT2 sulfate) was obtained. The water content, determined according to Karl Fischer was 2.5 %.

### Example 2

### Preparation of (3-(1-piperazinyl)1,2-benzisothiazole) sulfate hydrate (INT 2 sulfate)

An amount of 50 g of 3-(1-piperazinyl)-1,2-benzisothiazole (INT2), 500 ml of demineralized water and 33.5 g of H2SO4 (INT 2 : H2SO4 ratio is 1 : 1.5) were mixed at room temperature for 47 hour. After the completion of process clear solution became white. The product was filtered, washed with 125 ml demineralized water and dried for 2 hours at 30 C. An amount of 42.6 g of white precipitate of 3-(1-piperazinyl)-1,2-benzisothiazole sulfate hydrate (INT2 sulfate) was obtained.

### Example 3

### Preparation of 5- [2- [4- (1, 2- benzisothiazol-3-yl) -1-piperazinyl] ethyl] -6-chloro-1, 3- dihydro-2H-indol-2-one (Ziprasidone base)

In a round bottom flask 1 g of 6-Chloro-5-(2-Chloroethyl)-1,3-Dihydro-2H-Indol-2-One (INT 1), 1.1 g of 3-(1-piperazinyl)1,2-benzisothiazole (INT2), 1.46 g of Na2CO3 and 8 ml of 1-ethyl-3-methylimidazolium methyl sulfate were placed, heated to 100 °C and stirred for 24 hours. After the completion of the reaction the presence of 5- [2- [4- (1, 2- benzisothiazol-3-yl)-1-piperazinyl] ethyl] -6-chloro-1, 3- dihydro-2H-indol-2-one was confirmed by HPLC. The reaction mixture was cooled to room temperature and 30 ml of methanol was added to precipitate the product. Reaction mixture was stirred for about 1 h and filtered. An amount of 1.8 g of wet crude Ziprasidone base was obtained (Purity 88.0 % was determined by HPLC). A whole sample was further placed in a 50 ml round bottom flask with 20 ml of demineralised water and heated at the 79°C for 30 minutes, filtrated and cooled to room temperature. A 2.65 g of precipitated wet Ziprasidone base was dried in a vacuum drier for 1 h at the temperature 50 °C and overnight at the room temperature. An amount of 1.03 g of Ziprasidone base was obtained.

### Example 4

### Preparation of 5- [2- [4- (1, 2- benzisothiazol-3-yl) -1-piperazinyl] ethyl] -6-chloro-1, 3- dihydro-2H-indol-2-one (Ziprasidone base)

Example 2 was repeated with the exception that 1-butyl-3-methylimidazolium tetrafluoroborate was used instead of 1-ethyl-3-methylimidazolium methyl sulfate. After the completion of the reaction the presence of 5- [2- [4- (1, 2- benzisothiazol-3-yl) -1-piperazinyl] ethyl] -6-chloro-1, 3- dihydro-2H-indol-2-one was confirmed by HPLC.

### Example 5

### Preparation of 5- [2- [4- (1, 2- benzisothiazol-3-yl) -1-piperazinyl] ethyl] -6-chloro-1, 3-dihydro-2H-indol-2-one (Ziprasidone base)

Example 2 was repeated with the exception that 10 ml of 1-butyl-3-methylimidazolium bromide was used instead of 8 ml of 1-ethyl-3-methylimidazolium methyl sulfate. After the completion of the reaction the presence of 5- [2- [4- (1, 2- benzisothiazol-3-yl) -1-piperazinyl] ethyl] -6-chloro-1, 3- dihydro-2H-indol-2-one was confirmed by HPLC.

### Example 6

### Preparation of 5- [2- [4- (1, 2- benzisothiazol-3-yl) -1-piperazinyl] ethyl] -6-chloro-1, 3- dihydro-2H-indol-2-one hydrogensulfate dihydrate (Ziprasidone hydrogensulfate dihydrate)

In a round bottom flask 2 g of anhydrous (containing less than 1 % water defined by Karl-Fischer) 5- [2- [4- (1, 2- benzisothiazol-3-yl) -1-piperazinyl] ethyl] -6-chloro-1, 3- dihydro-2H-indol-2-one (Ziprasidon base), 20 ml demineralised water and 0.95 g of sulphuric acid were placed and mixed at the room temperature 15 to 60 minutes. A homogenous mixture was heated to 60 °C and intensively mixed for about 3 hours. The mixture was then cooled to room temperature, filtrated and rinsed with demineralsed water. Obtained product was dried in an air drier for about 2 hours at the 30°C until a constant weight was obtained. 2.46 g of the product was obtained. Water content was 7.1 % according to Karl-Fischer.

Solubility of the obtained Ziprasidone hydrogensulfate dihydrate was 0.36 mg/ml in water (pH 6.8) + 2 % SDS at room temperature. A two-week stability tests (40°C, humidity 75% and 50°C) show no degradation of the initial product ending with determined HPLC purity 99.7 % and 99.7 % of Ziprasidone, respectively.

### Example 7

### Preparation of 5- [2- [4- (1, 2- benzisothiazol-3-yl) -1-piperazinyl] ethyl] -6-chloro-1, 3- dihydro-2H-indol-2-one hydrogensulfate dihydrate (Ziprasidone hydrogensulfate dihydrate)

In a round bottom flask 23 g of anhydrous (containing less than 2 % water defined by Karl-Fischer) 5- [2- [4- (1, 2- benzisothiazol-3-yl) -1-piperazinyl] ethyl] -6-chloro-1, 3- dihydro-2H-indol-2-one (Ziprasidone base), 230 ml demineralised water and 11 g of sulphuric acid were placed and mixed at the room temperature 15 to 60 minutes. A homogenous mixture was heated to 60 °C and intensively mixed for about 3 hours. The mixture was then cooled to room temperature, filtrated and rinsed with demineralised water. Obtained product was dried in an air drier for about 2 hours at the 30°C until a constant weight was obtained. 29.9 g of the product was obtained. Water content was 6.8 % according to Karl-Fischer.

### Example 8

### Preparation of 5- [2- [4- (1, 2- benzisothiazol-3-yl) -1-piperazinyl] ethyl] -6-chloro-1, 3- dihydro-2H-indol-2-one hydrogensulfate dihydrate (Ziprasidone hydrogensulfate dihydrate)

In a round bottom flask 7.5 g of anhydrous (containing less than 1 % water defined by Karl-Fischer) 5- [2- [4- (1, 2- benzisothiazol-3-yl) -1-piperazinyl] ethyl] -6-chloro-1, 3- dihydro-2H-indol-2-one (Ziprasidone base), 75 ml demineralised water and 1.95 g of sulphuric acid were placed and mixed at the room temperature 15 to 60 minutes. A homogenous mixture was heated to 60 °C and intensively mixed for about 3 hours. The mixture was then cooled to room temperature, filtrated and rinsed with demineralised water. Obtained product was dried in an air drier for about 2 hours at the 30°C until a constant weight was obtained. 10.1 g of the product was obtained. Water content was 6.9 % according to Karl-Fischer.

### Example 9

### Preparation of 5- [2- [4- (1, 2- benzisothiazol-3-yl) -1-piperazinyl] ethyl] -6-chloro-1, 3- dihydro-2H-indol-2-one hydrogensulfate dihydrate (Ziprasidone hydrogensulfate anhydrous)

An amount of 300 mg of Ziprasidone hydrogensulfate dihydrate as obtained in Example 6 was dried in for 12 h at the temperature of 100 °C in an air drier. The water content of the obtained ziprasidone hydrogen sulfate anhydrous was less than 0.5 % according to Karl-Fischer.

### Example 10

### Preparation of 5- [2- [4- (1, 2- benzisothiazol-3-yl) -1-piperazinyl] ethyl] -6-chloro-1, 3- dihydro-2H-indol-2-one hydrogensulfate dihydrate (Ziprasidone hydrogensulfate anhydrous)

An amount of 1 g of ziprazidone hydrogensulfate dihydrate as obtained in example 6 was dried in for 12 h at the temperature in a vacuum drier. The water content of the obtained ziprazidone hydrogensulfate anhydrous was less than 0.5 % according to Karl Fischer.

### Example 11

An amount of 20 mg of zipasidone hydrogensulfate dihydrate was exposed to a watersorption analysis (Surface Measurement Systems Ltd., London, UK; two complete 11-step circles from 0 % RH to 95 % RH at the temperature 25°C and nitrogen flow rate 200 ml/min; minimal retention time at the step when dm/dt < 0.002% was 10 minutes and maximal 360 minutes) and compared to the same test of Ziprasidone hydrochloride monohydrate.

Ziprasidone hydrogensulfate salt was more stable to hydratation with the regard to Ziprasidone hydrochloride salt. Zipasidone hydrogensulfate dihydrate does not release crystal water at the 0 % RH at the performed test, comparing to Ziprasidone hydrochloride monohydrate which release crystal water at 0 % RH. Furthermore, water sorption of Ziprasidone hydrochloride started at 30 % RH, whereas water sorption of zipasidone hydrogensulfate started at 50 % RH.

### Example 12

### Formulation

All the compositions contain 35.26 Wt. % of Ziprasidone hydrogensulfate dihydrate, which was equivalent to 26.66 Wt. % of Ziprasidone free base. Particle size of the Ziprasidone hydrogensulfate dihydrate was 26 µm.

### Example 12. A

| Ingredients | Wt. % Composition |
|---|---|
| Ziprasidone hydrogensulfate dihydrate | 35.26 |
| Lactose Monohydrate | 54.48 |
| Pregelatinized Starch | 9.12 |
| Magnesium Stearate (I) | 0.91 |
| Magnesium Stearate (II) | 0.23 |
| Total | 100.0 |

Ziprasidone hydrogensulfate dihydrate capsules were manufactured by direct fill process. All the components were sieved and homogenized. The resulting blend was filled into size 2 hard gelatin capsules, each containing 300 mg of the composition.

The formulation can also be prepared using dry granulation process. In this case the Ziprasidone hydrogensulfate dihydrate was combined with lactose monohydrate, pregelatinized starch and magnesium stearate (I). The powder mixture was sieved, dry compacted and the agglomerates were milled. Then the magnesium stearate (II) was added and the resulting was filled into size 2 hard gelatine capsules, each containing 300 mg of the composition.

Another useful process of preparation was wet granulation. In the scope of this method, Ziprasidone hydrogensulfate dihydrate, lactose monohydrate and pregelatinized starch were sieved and blended. The powder mixture was granulated by adding purified water, while kneading the mixture. Drying of wet granulate was performed in the oven at 50°C for 4 hours. The magnesium stearate was added and the resulting blend was filled into size 2 hard gelatine capsules, each containing 300 mg of the composition.

### Example 12. B

| Ingredients | Wt. % Composition |
|---|---|
| Ziprasidone hydrogensulfate dihydrate | 35.26 |
| Lactose Monohydrate | 49.48 |
| Pregelatinized Starch | 9.12 |
| Polyethylene glycol 6000 | 5.0 |
| Magnesium Stearate | 1.14 |
| Total | 100.0 |

The pharmaceutical composition was prepared by wet granulation process at the batch size of 0.5 kg. Ziprasidone hydrogensulfate dihydrate, lactose monohydrate, pregelatinized starch and polyethylene glycol were sieved and blended. Granulation was performed by adding purified water, while kneading the mixture. The wet granulate was dried in the oven at 50°C for 4 hours. After magnesium stearate was added, the resulting blend was filled into size 2 hard gelatine capsules, each containing 300 mg of the composition.

### Example 12. C

| Ingredients | Wt. % Composition |
|---|---|
| Ziprasidone hydrogensulfate dihydrate | 35.26 |
| Lactose Monohydrate | 59.19 |
| Sodium starch glycolate | 3.97 |
| Sodium lauryl sulfate | 0.39 |
| Amorphous silica | 0.39 |
| Magnesium Stearate | 0.80 |
| Total | 100.0 |

Ziprasidone hydrogensulfate dihydrate capsules were manufactured by direct fill process. All the components were sieved and homogenized. The resulting blend was filled into size 2 hard gelatine capsules, each containing 300 mg of the composition.

### Example 13

### i. Synthesis of 5- [2- [4- (1, 2- benzisothiazol-3-yl) -1-piperazinyl] ethyl] -6-chloro-1, 3-dihydro-2H-indol-2-one (Ziprasidone base)

An amount of 5.00 g (0.023 mol) of 3-(1-piperazinyl)1,2-benzisothiazole, 5.77 g (0.025 mol) of 6-Chloro-5-(2-Chloroethyl)-1,3-Dihydro-2H-Indol-2-One, 2.44 g (0.023 mol) of Na₂CO₃ and 37.5 ml of 1-ethyl-3-methylimidazolium ethylsulfate were mixed to obtain homogenous suspension. The suspension was heated to about 100 °C and maintained at the same temperature until the end of reaction. The reaction mixture was then cooled to room temperature and precipitated with 50 ml of MeOH, mixed for 1 hour and filtrated. Finally, 10 g of product containing 97.6 area % (by HPLC) of Ziprasidone base were obtained.

### ii. Synthesis of 5- [2- [4- (1, 2- benzisothiazol-3-yl) -1-piperazinyl] ethyl] -6-chloro-1, 3-dihydro-2H-indol-2-one (Ziprasidone base)

Example 13.i. was repeated with the exception that 50 ml of 2-propanol were used instead of methanol for precipitation. 10.1 g of wet product containing 97.6 area % (by HPLC) of Ziprasidone base was obtained.

### iii. Synthesis of 5- [2- [4- (1, 2- benzisothiazol-3-yl) -1-piperazinyl] ethyl] -6-chloro-1, 3-dihydro-2H-indol-2-one (Ziprasidone base)

Filtrate (mixture of ionic liquid and methanol) as obtained according to Example 13.i. was reused for reaction in such way that methanol was evaporated. Example 13.i. was repeated with the exception that reused 1-ethyl-3-methylimidazolium ethylsulfate was added. Purity of the obtained product was > 90 area % (by HPLC).

### Example 14

### i. Purification of 5- [2- [4- (1, 2- benzisothiazol-3-yl) -1-piperazinyl] ethyl] -6-chloro-1, 3-dihydro-2H-indol-2-one (Ziprasidone base)

An amount of 18 kg of Ziprasidone base and 1.9 kg of active carbon were charged into 869 l of tetrahydrofurane under inert gas atmosphere and room temperature (approx. 22°C). The temperature was then increased to about 65 °C while mixing and maintained at the obtained temperature for max. 30 minutes and then filtered at about the same temperature at vacuum filter. The filtrate was concentrated to about 10 % of the initial volume under vacuum (less than about 50 mbar) and temperature 50°C in the reactor under the inert gas atmosphere. The concentrate was cooled to about 23°C to 27°C, pressure was increased to ambient pressure with inert gas, and 86.9 l of methanol was added. Precipitate was centrifuged and rinsed with 20 l of methanol and 40 l of demineralised water. All impurities were below 0.1 area % (by HPLC).

### ii. Maceration of 5- [2- [4- (1, 2- benzisothiazol-3-yl) -1-piperazinyl] ethyl] -6-chloro-1, 3-dihydro-2H-indol-2-one (Ziprasidone base)

An amount of 49 g of crude wet Ziprasidone base was charged to 730 ml demineralized water and agitated at room temperature for 30 minutes then heated to 85 °C and hot filtrated. The product was washed with 70 ml of demineralized water. Yield was 40 g of wet Ziprasidone base, which was then dried in vacuum dryer at elevated temperature until the level of water was below 1 %.

### Example 15

### Preparation of Ziprasidone sulfate monohydrate (not falling under the scope of the present invention

An amount of 188 g of pregranulated (Viani granulator, 2mm sieve) Ziprasidone base was slowly added to 1870 ml of demineralised water and mixed at the room temperature for about 30 minutes. Temperature was increased to 60 °C and 53.5 g of sulphuric acid at 50 °C was slowly added and the mixed at the temperature 60 °C for 3 hours. The mixture was then cooled to room temperature, mixed for 15 minutes, filtered and rinsed with demineralised water. Wet product was dried in air dryer at temperature up to 50 °C until the constant weight. 188.4 g of Ziprasidone sulfate monohydrate was obtained. Ziprasidone sulfate monohydrate was confirmed with potentiometric titration with 0.1 M TBOH. Water content was 7.0 ±0.5 % according to Karl-Fischer

### Example 16

### Preparation of Ziprasidone hydrogensulfate dihydrate

An amount of 11.5 kg of pregranulated Ziprasidone base (water content 17.5 %) was slowly added to sulphuric acid (2.9 kg H2SO4/95 l deminearlised water) water and mixed at the room temperature for about 30 minutes. Temperature was increased to about 60 °C and mixed at the temperature obtained for 3 hours. The mixture was then cooled to room temperature, mixed for 15 minutes, filtered and rinsed with demineralised water. Wet product was dried in air dryer at temperature up to 50 °C until the level of water was between 7.0 ±0.5 % according to Karl-Fisher. Ziprasidone hydrogensulfate dihydrate was confirmed with potentiometric titration with 0.1 M TBOH. 9.8 kg of Ziprasidone hydrogensulfate dihydrate was obtained.

### Example 17

### Preparation of Ziprasidone maleate monohydrate (not falling under the scope of the present invention)

An amount of 222 g of pregranulated Ziprasidone base was slowly added to 1900 ml of demineralised water and mixed at the room temperature for about 30 minutes. Temperature was increased to 60 °C and 125 g of maleic acid dissolved in 400 ml demineralised water at 50 °C was slowly added and the mixed at the temperature 60 °C for 3 hours. The mixture was then cooled to room temperature, mixed for 15 minutes, filtered and rinsed with demineralised water. Product was dried in vacuum dryer at temperatures up to 60 °C for 3 h. 292 g of Ziprasidone maleate monohydrate was obtained (purity > 99 % by HPLC). Water content was 2.3 % according to Karl-Fischer.

### Example 18

### Capsules containing Ziprasidone hydrogensulfate dihydrate

Capsules containing Ziprasidone hydrogensulfate dihydrate having compositions as listed in Table were prepared in the same manner as in Example 12.

| Composition | A | B | C | D | E |
|---|---|---|---|---|---|
| | % in the capsule (w/w) | | | | |
| Ziprasidone hydrogen sulfate dihydrate | 26.50 | 26.50 | 26.50 | 26.50 | 26.50 |
| Lactose monohydrate (100) | 60.45 | 60.95 | 51.20 | 52.00 | 60.45 |
| Starch 1500 (pregelatinized) | 10.00 | 10.00 | 9.00 | 9.00 | 10.00 |
| Maize starch | / | / | 12.50 | 12.50 | / |
| PVP K 25 | 2.55 | 2.55 | / | / | 2.05 |
| Ethanol (96 per cent) | q.s. | q.s. | / | / | / |
| Purified water | / | / | q.s. | q.s. | / |
| Magnesium Stearate | 0.50 | / | 0.80 | / | 1.00 |
| Total | 100.0 | 100.0 | 100.0 | 100.0 | 100.0 |

Granulate/encapsulating mixture was then filled into hard gelatin capsules:

| Dose (mg) | Size | Cap | | Body | |
|---|---|---|---|---|---|
| 20 | 4 | 17.090 LT GREEN OP.C090 | | L500 WHITE | |
| | | Indigo carmine-FD&C Blue 2 | 0.0170 % | Titanium dioxide | 2.0000 % |
| | | Titanium dioxide | 2.0000 % | Gelatin q.s.p | 100 % |
| | | Yellow iron oxide | 0.2500 % | | |
| | | Gelatin q.s.p | 100 % | | |
| 40 | 3 | 18.385 DARK GREEN OP.C385 | | 17.090 LT GREEN OP.C090 | |
| | | Indigo carmine-FD&C Blue 2 | 0.0890 % | Indigo carmine-FD&C Blue 2 | 0.0170 % |
| | | Titanium dioxide | 0.7418 % | Titanium dioxide | 2.0000 % |
| | | Yellow iron oxide | 0.6120 % | Yellow iron oxide | 0.2500 % |

| | | Gelatin q.s.p | 100 % | Gelatin q.s.p | 100 % |
|---|---|---|---|---|---|
| 60 | 2 | 18.385 DARK GREEN OP.C385 | | L500 WHITE | |
| | | Indigo carmine-FD&C Blue 2 | 0.0890 % | Titanium dioxide | 2.0000 % |
| | | Titanium dioxide | 0.7418 % | Gelatin q.s.p | 100 % |
| | | Yellow iron oxide | 0.6120 % | | |
| | | Gelatin q.s.p | 100 % | | |
| 80 | 1 | 17.090 LT GREEN OP.C090 | | L500 WHITE | |
| | | Indigo carmine-FD&C Blue 2 | 0.0170% | Titanium dioxide | 2.0000 % |
| | | Titanium dioxide | 2.0000 % | Gelatin q.s.p | 100 % |
| | | Yellow iron oxide | 0.2500 % | | |
| | | Gelatin q.s.p | 100 % | | |

### Example 19

### Preparation of Ziprasidone sulfate of form II

To an amount of 2 g of Ziprasidone base 20 ml of demineralised water was added. Temperature was increased to 75-80 °C while mixing. Then 0,62 g of sulphuric acid was slowly added in 15 min at the same conditions (mixing and the temperature maintained at 75-80°C). After the addition of sulphuric acid the reaction temperature was decreased to 60 °C and mixed for another 3 hours. The mixture was then cooled to room temperature, mixed for 15 minutes, filtered and rinsed with demineralised water. Wet product was dried in air dryer at temperature up to 50 °C until the constant weight.

Obtained product was characterised by X-ray powder diffraction pattern exhibiting characteristic diffraction angles 7,7; 12,4; 14,4; 15,5; 19,4; 20,1 and 27,1 ± 0.2°Theta.

### Example 20

### Capsules containing milled Ziprasidone hydrogensulfate dihydrate

Ziprasidone hydrogensulfate dihydrate having particle size 64 µm, 115 µm and 100 µm were milled at Hosokawa PK 57.01 jet mill (3000 rpm) and particle size of 13 µm, 15 µm and 18 µm were obtained, respectively.

Capsules containing ziprasidone hydrogensulfate dihydrate having compositions as listed in Example 18 were prepared in the same manner, wherein milled ziprasidone hydrogen-sulphate dihydrate of particle size 13 µm, 15 µm and 18 µm were used.

## Claims

1. Compound of formula I:
[Ziprasidone ∗ H]ₙ⁺ [X] ∗ Z H₂O
wherein
Ziprasidone is a compound of the formula; 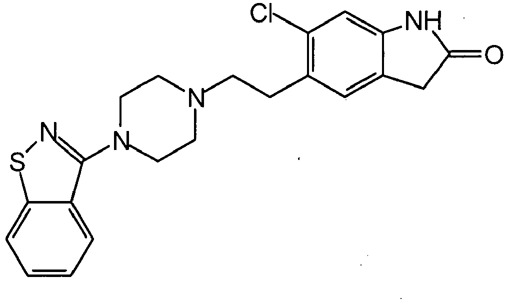
H is hydrogen;
n is 1 or 2;
X is HSO₄⁻ or SO₄²⁻; and
Z is 2.

2. The compound according to claim 1, wherein said compound has a particle size in the range of about 5 µm to about 300 µm.

3. The compound according to any of the proceeding claims, wherein said compound has a solubility in the range of about 50 mg/l to about 800 mg/l.

4. The compound according to claim 1,
**characterized by** a X-ray powder diffraction pattern exhibiting characteristic diffraction angles at 8.6; 12.1; 13.6; 17.6; 19.8; 23.4 and 24.3° ± 0.2° Theta; or **characterized by** a X-ray powder diffraction pattern exhibiting characteristic diffraction angles at 4.5; 9.0; 15.6; 23.7; 25.0 and 26.6° ± 0.2°2 Theta.

5. A process for the preparation of a compound of formula I according to claim 1, comprising the steps of:
- providing Ziprasidone base;
- reacting with water and sulfuric acid;
- optionally cooling and/or drying; and
- obtaining compound of formula I.

6. The process according to claim 5, wherein anhydrous Ziprasidone base is provided by reacting 6-Chloro-5-(2-Chloroethyl)-1,3-Dihydro-2H-Indol-2-One and 3-(1-piperazinyl)-1,2-benzisothiazole in presence of an alkaline compound and an organic solvent, optionally mixed with water.

7. The process according to claim 6, wherein the alkaline compound is Na₂CO₃.

8. The process according to claim 6, wherein the organic solvent is an ionic liquid.

9. The process according to claim 8, wherein the ionic liquid selected from the group 1-R-3-R1-imidazolium H(+) x A(-), wherein R and R1 are lower alkyl and A(-) are mono- or polyprotic anion of acid.

10. Use of a compound according to any of the claims 1-4 for the preparation of a pharmaceutical composition for the prevention and/or treatment of psychotic states.

11. The use according to claim 10, wherein the psychotic states include schizophrenia and anxiety diseases.

12. (3-(1-piperazinyl)1,2-benzisothiazole) sulfate hydrate **characterized by** a X-ray powder diffraction pattern exhibiting characteristic diffraction angles at 12.9; 14.0; 17.1; 19.6; 21.0; 22.3 and 26.7° ± 0,2° Theta.

## Patentansprüche

1. Verbindung der Formel I:
[Ziprasidon ∗ H]ₙ⁺ [X] ∗ Z H₂O
worin
Ziprasidon eine Verbindung der Formel ist; 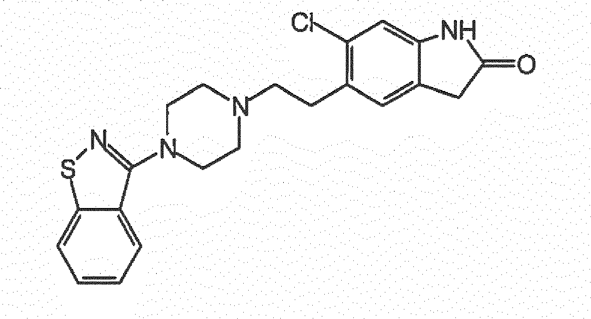
H Wasserstoff ist;
n 1 oder 2 ist;
X HSO₄⁻ oder SO₄²⁻ ist; und
Z 2 ist.

2. Verbindung nach Anspruch 1, worin die Verbindung eine Teilchengröße im Bereich von etwa 5 µm bis etwa 300 µm aufweist.

3. Verbindung nach einem der vorstehenden Ansprüche, worin die Verbindung eine Löslichkeit im Bereich von etwa 50 mg/l bis etwa 800 mg/l aufweist.

4. Verbindung nach Anspruch 1,
**gekennzeichnet durch** ein Röntgen-Pulverbeugungsdiagramm mit charakteristischen Beugungswinkeln bei 8,6; 12,1; 13,6; 17,6; 19,8; 23,4 und 24,3° ± 0.2° Theta; oder **gekennzeichnet durch** ein Röntgen-Pulverbeugungsdiagramm mit charakteristischen Beugungswinkeln bei 4,5; 9,0; 15,6; 23,7; 25,0 und 26,6° ± 0.2° Theta.

5. Verfahren zur Herstellung einer Verbindung der Formel I nach Anspruch 1, welches die Schritte umfasst:
- Bereitstellen von Ziprasidon-Base;
- Umsetzen mit Wasser und Schwefelsäure;
- wahlweise Kühlen und/oder Trocknen; und
- Erhalten der Verbindung der Formel I.

6. Verfahren nach Anspruch 5, worin wasserfreie Ziprasidon-Base bereitgestellt wird durch Umsetzen von 6-Chlor-5-(2-chlorethyl)-1,3-dihydro-2H-indol-2-on und 3-(1-Piperazinyl)-1,2-benzisothiazol in Anwesenheit einer alkalischen Verbindung und eines organischen Lösungsmittels, wahlweise gemischt mit Wasser.

7. Verfahren nach Anspruch 6, worin die alkalische Verbindung Na₂CO₃ ist.

8. Verfahren nach Anspruch 6, worin das organische Lösungsmittel eine ionische Flüssigkeit ist.

9. Verfahren nach Anspruch 8, worin die ionische Flüssigkeit ausgewählt ist aus der Gruppe von 1-R-3-R1-Imidazolium H(+) x A(-), worin R and R1 niederes Alkyl und A(-) mono- or polyprotische Anionen einer Säure sind.

10. Verwendung einer Verbindung nach einem der Ansprüche 1-4 zur Herstellung einer pharmazeutischen Zusammensetzung zur Verhinderung und/oder Behandlung von psychotischen Zuständen.

11. Verwendung nach Anspruch 10, worin die psychotischen Zustände Schizophrenie und Angst-Erkrankungen umfassen.

12. (3-(1-Piperazinyl)1,2-benzisothiazol)sulfat-Hydrat, **gekennzeichnet durch** ein Röntgen-Pulverbeugungsdiagramm mit charakteristischen Beugungswinkeln bei 12,9; 14,0; 17,1; 19,6; 21,0; 22,3 und 26,7° ± 0,2° Theta.

## Revendications

1. Composé de formule I :
[Ziprasidone ∗ H]ₙ⁺ [X] ∗ Z H₂O
ziprasidone étant un composé de la formule ; 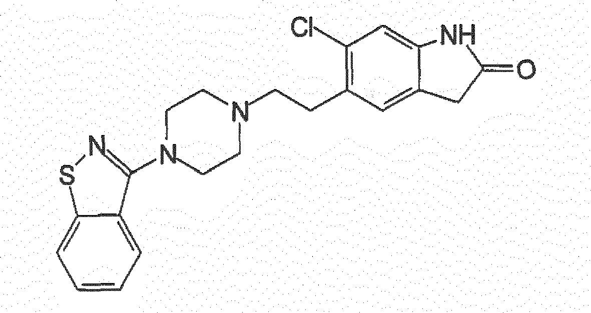
H représentant hydrogène ;
n valant 1 ou 2 ;
X représentant HSO₄⁻ ou SO₄²⁻ ; et
Z représentant 2

2. Composé selon la revendication 1, ledit composé présentant une grosseur de particule dans la plage d'environ 5 µm à environ 300 µm.

3. Composé selon l'une quelconque des revendications précédentes, ledit composé présentant une solubilité dans la plage d'environ 50 mg/l à environ 800 mg/l.

4. Composé selon la revendication 1,
**caractérisé par** un diagramme de diffraction de rayons X sur poudre présentant des angles de diffraction caractéristiques à 8,6 ; 12,1 ; 13,6 ; 17,6 ; 19,8 ; 23,4 et 24,3° ± 0,2° Théta ; ou **caractérisé par** un diagramme de diffraction de rayons X sur poudre présentant des angles de diffraction caractéristiques à 4,5 ; 9,0 ; 15,6 ; 23,7 ; 25,0 et 26,6° ± 0,2° 2 Thêta.

5. Procédé de préparation d'un composé de formule I selon la revendication 1, comprenant les étapes :
- de mise à disposition d'une base de Ziprasidone ;
- de réaction avec de l'eau et de l'acide sulfurique ;
- éventuellement de refroidissement et/ou de séchage ; et
- d'obtention d'un composé de la formule I.

6. Procédé selon la revendication 5, la base de ziprasidone anhydre étant obtenue par réaction de 6-chloro-5-(2-chloroéthyl)-1,3-dihydro-2H-indol-2-one et de 3-(1-pipérazininyl)-1,2-benzisothiazole en présence d'un composé alcalin et d'un solvant organique, éventuellement mélangé à de l'eau.

7. Procédé selon la revendication 6, le composé alcalin étant du Na₂CO₃.

8. Procédé selon la revendication 6, le solvant organique étant un liquide ionique.

9. Procédé selon la revendication 8, le liquide ionique étant sélectionné dans le groupe 1-R-3-R1-imidazolium H(+) x A(-), R et R1 représentant alkyle inférieur et A(-) représentant un anion d'acide monoprotique ou polyprotique.

10. Utilisation d'un composé selon l'une quelconque des revendications 1 à 4 pour la préparation d'une composition pharmaceutique pour la prévention et/ou le traitement d'états psychotiques.

11. Utilisation selon la revendication 10, les états psychotiques comprenant la schizophrénie et les maladies de l'anxiété.

12. Sulfate de (3-(1-pipérazinyl)1,2-benzisothiazole) hydraté **caractérisé par** un diagramme de diffraction de poudre de rayons X présentant des angles de diffraction caractéristiques à 12,9 ; 14,0 ; 17,1 ; 19,6 ; 21,0 ; 22,3 et 26,7° ± 0,2° Thêta.
